(19) Europäisches Patentamt
European Patent Office
Office européen des brevets

(11) Veröffentlichungsnummer: **0 288 960 A1**

(12) **EUROPÄISCHE PATENTANMELDUNG**

(21) Anmeldenummer: **88106619.5**

(51) Int. Cl.⁴: **C07D 209/48 , A01N 37/32**

(22) Anmeldetag: **26.04.88**

Patentansprüche für folgenden Vertragsstaat: ES.

(30) Priorität: **30.04.87 DE 3714372**

(43) Veröffentlichungstag der Anmeldung:
**02.11.88 Patentblatt 88/44**

(84) Benannte Vertragsstaaten:
**AT BE CH DE ES FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Liebl, Rainer, Dr.**
**An der Weinleite 5b**
**D-8901 Todtenweis(DE)**
Erfinder: **Bauer, Klaus, Dr.**
**Doorner Strasse 53D**
**D-6450 Hanau(DE)**
Erfinder: **Bieringer, Hermann, Dr.**
**Eichenweg 26**
**D-6239 Eppstein/Taunus(DE)**

(54) **Alkyl-N-aryl-tetrahydrophthalimide, Verfahren zu ihrer Herstellung sowie ihre Verwendung im Pflanzenschutz.**

(57) Verbindungen der Formel I

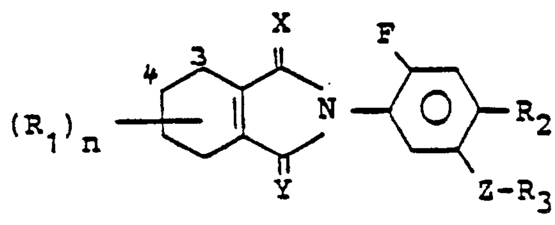

(I)

worin
X, Y, Z = O oder S; $R_1$ Alkyl, $R_2$ Fluor, Chlor, Brom oder $NO_2$; $R_3$ (subst.) Alkyl, Cycloalkyl, Alkenyl, Alkinyl, (subst.) Phenyl oder einen Rest der Formeln

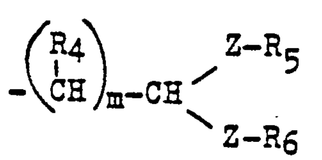

,

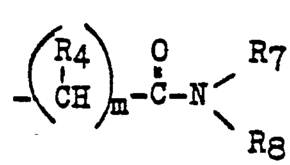

oder

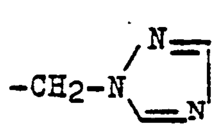

,

EP 0 288 960 A1

$R_4$ Wasserstoff oder Alkyl; $R_5$, $R_6$ oder beide Reste zusammen eine ($C_2$-$C_3$)Alkylenkette; $R_7$, $R_8$ Wasserstoff, Alkyl, (subst.) Phenyl oder (subst.) Benzyl, oder $R_7$ und $R_8$ bilden zusammen mit dem sie verknüpfenden N-Atom einen 5-oder 6-gliedrigen gesättigten (subst.) Heterocyclus, der als Ringglied auch ein Sauerstoffatom oder eine Gruppe N-$R_4$ enthalten kann,
n 1 oder 2 und m 1, 2 oder 3 bedeuten, besitzen vorteilhafte herbizide Eigenschaften.

# Alkyl-N-aryl-tetrahydrophthalimide, Verfahren zu ihrer Herstellung sowie ihre Verwendung im Pflanzenschutz

Bestimmte Alkyl-N-aryl-tetrahydrophthalimide sowie deren herbizide Wirksamkeit sind in DE-A 3 109 035 (US-A-4,594,099) und JP-A 49,006,121 beschrieben.

Weiterhin ist die herbizide Wirksamkeit von bestimmten N-Aryl-tetrahydrophthalimiden bekannt, die keine Alkylgruppe im Phthalimidrest besitzen, s. EP-A 095 192.

Es wurden neue Alkyl-N-aryl-tetrahydrophthalimide mit ausgewähltem Substitutionsmuster im Arylteil gefunden, die überraschenderweise deutlich höhere herbizide Wirkungen besitzen und gleichzeitig eine hervorragende Verträglichkeit gegenüber Kulturpflanzen aufweisen.

Gegenstand der vorliegenden Erfindung sind daher die Tetrahydrophthalimide der Formel I,

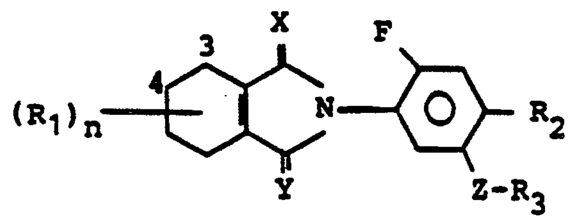

(I)

worin

X,Y,Z unabhängig voneinander O oder S,

$R_1$ $(C_1\text{-}C_4)$Alkyl,

$R_2$ Fluor, Chlor, Brom oder $NO_2$

$R_3$ $(C_1\text{-}C_4)$Alkyl, Halogen$(C_1\text{-}C_4)$alkyl, $(C_3\text{-}C_6)$Cycloalkyl, $(C_3\text{-}C_4)$Alkenyl, $(C_3\text{-}C_4)$Alkinyl, Cyano$(C_1\text{-}C_4)$alkyl, $(C_1\text{-}C_4)$Alkoxy-$(C_1\text{-}C_4)$alkyl, $(C_1\text{-}C_4)$Alkylthio$(C_1\text{-}C_4)$alkyl, $(C_1\text{-}C_4)$Alkoxycarbonyl$(C_1\text{-}C_4)$alkyl, wobei der Alkoxyteil bis zu 3-fach durch Halogen substituiert sein kann,
$(C_1\text{-}C_4)$Alkylthiocarbonyl$(C_1\text{-}C_4)$alkyl, $(C_1\text{-}C_4)$Alkoxythiocarbonyl$(C_1\text{-}C_4)$alkyl, $(C_1\text{-}C_4)$Alkoxy$(C_1\text{-}C_4)$-alkoxycarbonyl$(C_1\text{-}C_4)$alkyl, $(C_1\text{-}C_4)$Alkoxycarbonyl$(C_1\text{-}C_4)$alkoxycarbonyl$(C_1\text{-}C_4)$alkyl,
Phenyl oder Phenyl$(C_1\text{-}C_4)$alkyl, wobei der Phenylring der beiden Reste bis zu 3-fach durch Halogen, $(C_1\text{-}C_4)$Alkyl, $(C_1\text{-}C_4)$Alkoxy, $(C_1\text{-}C_4)$Alkoxycarbonyl, $CF_3$, $NO_2$ oder CN substituiert sein kann, oder
einen Rest der Formeln

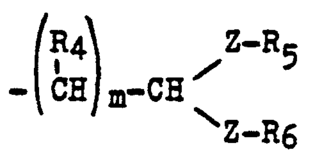

,

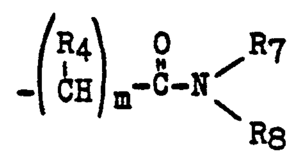

oder

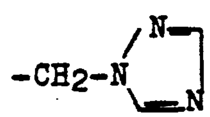

$R_4$ Wasserstoff oder $(C_1\text{-}C_4)$Alkyl,

$R_5$, $R_6$ $(C_1\text{-}C_4)$Alkyl oder beide Reste zusammen eine Ethylen-oder Propylen-Kette,

$R_7$, $R_8$ unabhängig voneinander Wasserstoff, $(C_1\text{-}C_4)$Alkyl, Phenyl oder Benzyl, die beide im Phenylteil bis zu zweifach durch $(C_1\text{-}C_4)$Alkyl, Halogen, $(C_1\text{-}C_4)$Alkoxy, $CF_3$, $NO_2$ oder CN substituiert sein können; oder $R_7$ und $R_8$ bilden zusammen mit dem sie verknüpfenden N-Atom einen 5-oder 6-gliedrigen gesättigten Heterocyclus, der als Ringglied auch ein Sauerstoffatom oder eine Gruppe $>N\text{-}R^4$ enthalten kann und der bis zu zweifach durch $(C_1\text{-}C_4)$Alkyl an den $CH_2$-Ringgliedern substituiert sein kann,

n 1 oder 2 und

m 1, 2 oder 3

bedeuten.

Alkoxy, Alkyl, Alkenyl oder Alkinyl kann in obigen Definitionen geradkettig oder verzweigt sein.

Als Heterocyclen für den Rest $-NR_7R_8$ sind insbesondere von Bedeutung Pyrrolidin, Piperidin, Piperazin, Morpholin, oder 2,6-Dimethylmorpholin. Halogen($C_1$-$C_4$)alkyl kann bis zu sechs Halogen-, insbesondere Fluor-oder Chloratome, enthalten und steht beispielsweise für 2,2,2-Trifluorethoxy, 1,1,2,2-Tetrafluorethoxy, 1,1,2-Trifluor-2-chlor-ethoxy oder auch 1,1,2,3,3,3-Hexafluorpropoxy.

Als bevorzugt sind die Verbindungen der Formel I anzusehen, bei denen X, Y Sauerstoff, $R_1$ in Position 4 orientiert ist und Methyl, $R_2$ Chlor oder Brom, $R_3$ ($C_1$-$C_4$)Alkyl, Halogen($C_1$-$C_4$)-alkyl, ($C_3$-$C_4$)Alkenyl; ($C_3$-$C_4$)Alkinyl; ($C_1$-$C_4$)Alkoxycarbonyl($C_1$-$C_4$)alkyl; Cyano($C_1$-$C_4$)Alkyl; Benzyl, das bis zu zweifach durch Halogen, ($C_1$-$C_4$)Alkyl, ($C_1$-$C_4$)Alkoxy oder $CF_3$ substituiert ist oder ein Rest der Formel

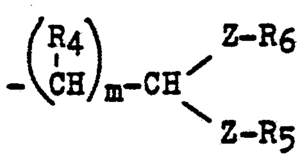 ,
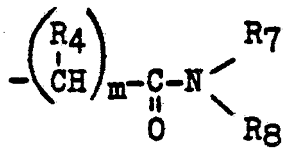 oder

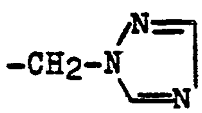

n = 1 und m = 1 oder 2 bedeuten.

Für $R_3$ ist insbesondere von Bedeutung ($C_1$-$C_4$)Alkyl, ($C_3$-$C_4$)Alkenyl, ($C_3$-$C_4$)Alkinyl, ($C_1$-$C_4$)-Alkoxycarbonyl-($C_1$-$C_4$)alkyl, Cyano($C_1$-$C_4$)alkyl oder Benzyl, das bis zu 2-fach durch Halogen oder ($C_1$-$C_4$)-Alkyl substituiert sein kann.

Ein weiterer Gegenstand der Erfindung sind Verfahren zur Herstellung von Verbindungen der allgemeinen Formel I, dadurch gekennzeichnet, daß man

a) eine Verbindung der Formel (II) mit einer Verbindung der Formel (III)

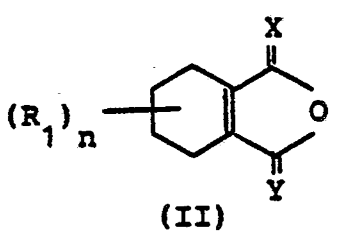
(II)

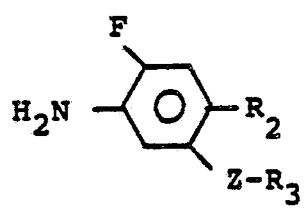
(III)

in Gegenwart von Wasser oder einem organischen Lösungsmittel, oder

b) eine Verbindung der Formel (IV)

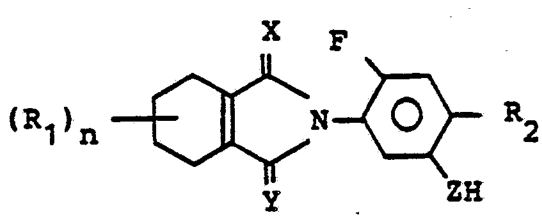

$R_3$-Hal
(V)

mit einer Verbindung der Formel (V) in Gegenwart einer Base und eines organischen Lösungsmittels umsetzt.

Als organische Lösungsmittel beim Verfahren a) können beispielsweise aromatische Lösungsmittel wie Toluol oder Xylol, Eisessig oder Dimethylformamid verwendet werden. Die Reaktionstemperatur variiert im allgemeinen zwischen 60° und 140°C.

Die Umsetzung kann auch durch saure Katalysatoren wie z.B. Schwefelsäure oder auch p-Toluolsulfonsäure, oder durch basische Katalysatoren wie z.B. Pyridin oder Triethylamin, beschleunigt werden.

Bei der Verfahrensvariante b) werden als Lösungsmittel beispielsweise aromatische Lösungsmittel wie Toluol, ferner Aceton, Acetonitril, Dimethylsulfoxid oder Dimethylformamid eingesetzt. Als Basen finden

anorganische Basen z.B. Alkalimetallhydroxide oder -carbonate wie NaOH, $K_2CO_3$ oder organische Basen wie Trialkylamine, Pyridin und ähnliche Verwendung.

Die Reaktionstemperaturen variieren im allgemeinen zwischen 40°C und 120°C.

Die Tetrahydrophthalsäureanhydride der allgemeinen Formel II sind z.T. bekannt, s. J. Org. Chem. 12 (1947) 713; J. Am. Chem. Soc. 72 (1950) 3732 oder lassen sich nach den dort beschriebenen Verfahren herstellen.

Die Verbindungen der Formel IV werden durch Umsetzung von Verbindungen der Formel II mit Verbindungen der Formel VI

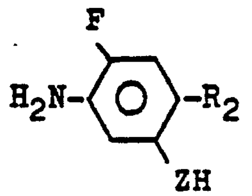

(VI)

erhalten

Die Umsetzung erfolgt analog der für a) beschriebenen Verfahrensweise.

Verbindungen der Formel III und VI werden nach dem Fachmann geläufigen Synthesemethoden hergestellt.

Die erfindungsgemäßen Verbindungen der Formel I weisen eine ausgezeichnete herbizide Wirksamkeit gegen ein breites Spektrum wirtschaftlich wichtiger mono-und dikotyler Schadpflanzen auf. Auch schwer bekämpfbare perennierende Unkräuter, die aus Rhizomen, Wurzelstöckchen oder anderen Dauerorganen austreiben, werden durch die Wirkstoffe gut erfaßt. Beispiele für solche Schadpflanzen sind verschiedene Arten von Avena, Lolium, Alopecurus, Phalaris, Echinochloa, Digitaria, Setaria, Cyperus, Agropyron, Cynodon, Imperata, Sorghum, Galium, Viola, Veronica, Lamium, Stellaria, Amaranthus, Sinapis, Ipomoea, Matricaria, Abutilon, Sida, Convolvulus, Cirsium, Rumex, Artemisia, Sagittaria, Alisma, Eleocharis und Scirpus. Dabei ist es gleichgültig, ob die Substanzen im Vorsaat-, Vorauflauf-oder Nachauflaufverfahren ausgebracht werden.

Werden die erfindungsgemäßen Verbindungen vor dem Keimen auf die Erdoberfläche appliziert, so wird entweder das Auflaufen der Unkrautkeimlinge vollständig verhindert, oder die Unkräuter wachsen bis zum Keimblattstadium heran, stellen jedoch dann ihr Wachstum ein und sterben schließlich nach Ablauf von drei bis vier Wochen vollkommen ab. Bei Applikation der Wirkstoffe auf die grünen Pflanzenteile im Nachauflaufverfahren tritt ebenfalls sehr rasch nach der Behandlung ein drastischer Wachstumsstop ein, und die Unkrautpflanzen bleiben in dem zum Applikationszeitpunkt vorhandenen Wuchsstadium stehen oder sterben nach einer gewissen Zeit mehr oder weniger schnell ab, so daß auf diese Weise eine für die Kulturpflanzen schädliche Unkrautkonkurrenz sehr früh und nachhaltig durch den Einsatz der neuen erfindungsgemäßen Verbindungen beseitigt werden kann.

Obgleich die erfindungsgemäßen Verbindungen eine ausgezeichnete herbizide Aktivität gegenüber mono-und dikotylen Unkräutern aufweisen, werden Kulturpflanzen wirtschaftlich bedeutender Kulturen wie z.B. Weizen, Gerste, Roggen, Reis, Mais, Zuckerrüben, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Däruber hinaus weisen die erfindungsgemäßen Verbindungen wachstumsregulatorische Eigenschaften bei Kulturpflanzen auf. Sie greifen regulierend in den pflanzeneigenen Stoffwechsel ein und können damit zur Ernteerleichterung wie z.B. durch Auslösen von Desikkation, Abszission und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono-und dikotylen Kulturen eine große Rolle, da das Lagern hierdurch verringert oder völlig verhindert werden kann.

Die erfindungsgemäßen Mittel können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem Wirkstoff außer gegebenenfalls einem Verdünnungs-oder Inertstoff noch Netzmittel, z.B. polyoxethylierte Alkylphenole, polyoxethylierte Fettalkohole, Alkyl-oder Alkylphenylsulfonate und Dispergiermittel, z.B. ligninsulfonsaures Natrium, 2,2′-dinaphthylmethan-6,6′-disulfonsaures Natrium, dibutylnaphthalinsulfonsaures Natrium oder

auch oleoylmethyltaurinsaures Natrium enthalten. Die Herstellung erfolgt in üblicher Weise, z.B. durch Mahlen und Vermischen der Komponenten.

Emulgierbare Konzentrate können z.B. durch Auflösen des Wirkstoffes in einem inerten organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden: Alkylarylsulfonsaure Calciumsalze wie Ca-dodecylbenzolsulfonat oder nichtionische Emulgatoren wie Fettsäurepolyglykolester, Alkyl-arylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel kann man durch Vermahlen des Wirkstoffes mit feinverteilten, festen Stoffen z.B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentrationen mittels Bindemitteln, z.B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise, gewünschtenfalls in Mischung mit Düngemitteln, granuliert werden.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll-oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. Sie kann innerhalb weiter Grenzen schwanken, z.B. zwischen 0,005 und 10,0 kg/ha oder mehr Aktivsubstanz, vorzugsweise liegt sie jedoch zwischen 0,01 und 5 kg/ha.

Auch Mischungen oder Mischformulierungen mit anderen Wirkstoffen, wie z.B. Insektiziden, Akariziden, Herbiziden, Düngemitteln, Wachstumsregulatoren oder Fungiziden sind gegebenenfalls möglich.

Die Erfindung wird durch nachstehende Beispiele näher erläutert.

Formulierungsbeispiele

A. Ein Stäubemittel wird erhalten, indem man 10 Gewichtsteile Wirkstoff und 90 Gewichtsteile Talkum oder Inertstoff mischt und in einer Schlagmühle zerkleinert.

B. Ein in Wasser leicht dispergierbares, benetzbares Pulver wird erhalten, indem man 25 Gewichtsteile Wirkstoff, 64 Gewichtsteile kaolinhaltigen Quarz aus Inerstoff, 10 Gewichtsteile ligninsulfonsaures Kalium und 1 Gewichtsteil oleoylmethyltaurinsaures Natrium als Netz-und Dispergiermittel mischt und in einer Stiftmühle mahlt.

C. Ein in Wasser leicht dispergierbares Dispersionskonzentrat wird erhalten, indem man 20 Gewichtsteile Wirkstoff mit 6 Gewichtsteilen Alkylphenolpolyglykolether (Triton x 207), 3 Gewichtsteilen Isotridecanolpolyglykolether (8 AeO) und 71 Gewichtsteilen paraffinischem Mineralöl (Siedebereich z.B. ca. 255 bis über 377°C) mitscht und in einer Reibkugelmühle auf eine Feinheit von unter 5 Mikron vermahlt.

D. Ein emulgierbares Konzentrat wird erhalten aus 15 Gewichtsteilen Wirkstoff, 75 Gewichtsteilen Cyclohexanon als Lösungsmittel und 10 Gewichtsteilen oxethyliertes Nonylphenol (10 AeO) als Emulgator.

Chemische Beispiele

Beispiel 1

N-(4-Chlor-2-fluor-5-methoxy-phenyl)-4-methyl-3,4,5,6-tetrahydrophthalimid

Man löst 8,3 g (0,05 mol) 4-Methyl-3,4,5,6-tetrahydrophthalsäureanhydrid und 8,8 g (0,05 mol) 4-Chlor-2-fluor-5-methoxy-anilin in 120 ml Eisessig und rührt 4 h bei 100°C.
Man gießt anschließend auf 300 ml Wasser, saugt den ausgefallenen Niederschlag ab und kristallisiert aus Ethanol um.
Man erhält 11,7 g (72 % d.Th.) N-(4-Chlor-2-fluor-5-methoxy-phenyl-)-4-methyl-3,4,5,6-tetrahydrophthalimid in Form farbloser Kristalle mit Schmp. 107 - 110°C.

Beispiel 2

N-(4-Chlor-2-fluor-5-propargyloxy-phenyl)-4-methyl-3,4,5,6-tetrahydrophthalimid

8,3 g (0.05 mol) 4-Methyl-3,4,5,6-tetrahydrophthalsäureanhydrid und 9,1 g (0,05 mol) 4-Chlor-2-fluor-5-hydroxy-anilin werden in 100 ml Eisessig 3 h bei 100°C gerührt.
Man gießt auf 300 ml Wasser und saugt den ausgefallenen Niederschlag ab.
Dieser wird ohne weitere Reinigung in 130 ml DMF gelöst. Man gibt 8,3 g (0,06 mol) Kaliumcarbonat und 6,0 g (0,05 mol) Propargylbromid dazu und rührt 5 h bei 70°C.
Man gießt auf 400 ml Wasser und saugt den ausgefallenen Niederschlsg ab. Nach der Umkristallisation aus Ethanol erhält man 14,3 g (82 % d. Th.) N-(4-Chlor-2-fluor-5-propargyloxy-phenyl)-4-methyl-3,4,5,6-tetrahydrophthalimid in Form hellbeiger Kristalle mit Schmp. 104 - 106°C.

Beispiel 3

N-(4-Chlor-2-fluor-5-methoxycarbonylmethoxy-phenyl)-4-methyl-3,4,5,6-tetrahydrophthalimid

8,3 g (0,05 mol) 4-Methyl-3,4,5,6-tetrahydrophthalsäureanhydrid und 11,7 g (0,05 mol) 4-Chlor-2-fluor-5-methoxycarbonylmethoxy-anilin werden in 150 ml Xylol gelöst. Nach Zugabe von 1 ml Triethylamin wird am Wasserabscheider bis zur beendeten Wasserbildung erhitzt.
Man wäscht mit 100 ml 2-normaler Salzsäure und anschließend mit 100 ml Wasser, trocknet die organische Phase über Natriumsulfat und entfernt das Lösungsmittel durch Destillation unter vermindertem Druck. Nach dem Trocknen am Hochvakuum erhält man 17,4 g (91 % d.Th) N-(4-Chlor-2-fluor-5-methoxycarbonylmethoxy-phenyl)-4-methyl-3,4,5,6-tetrahydrophthalimid in Form eines hellbraunen Sirups.
In analoger Weise lassen sich die Verbindungen der nachfolgenden Tabelle 1 erhalten.

Tabelle 1

| Beispiel | $R_1$ | $R_2$ | $R_3$ | Z | X | Y | Fp [°C] |
|---|---|---|---|---|---|---|---|
| 4 | $-CH_3$ | -Br | $-CH_2-C\equiv CH$ | O | O | O | Sirup |
| 5 | $-CH_3$ | -Cl | $-CH_2-CH=CH_2$ | O | O | O | Sirup |
| 6 | $-CH_3$ | -Cl | $-CH_2-$ 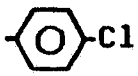 -Cl | O | O | O | 107 - 109 |
| 7 | $-CH_3$ | -Cl | $-CH-CO_2C_2H_5$<br>$CH_3$ | O | O | O | Sirup |
| 8 | $-CH_3$ | -Br | $-CH_2-CN$ | O | O | O | |
| 9 | $-CH_3$ | -Cl | $-CH_2-CN$ | O | O | O | |
| 10 | $-CH_3$ | -Br | $-CH_2-CH(OC_2H_5)_2$ | O | O | O | |
| 11 | $-CH_3$ | -Br | $-CH_2-CH$ 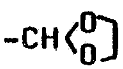 | O | O | O | |
| 12 | $-CH_3$ | -Br | $-CH_2-CH$ 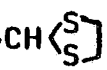 | O | O | O | |
| 13 | $-CH_3$ | -Br | $-CH(CH_3)_2$ | O | O | O | |
| 14 | $-CH_3$ | -Cl | $-CH(CH_3)_2$ | O | O | O | |
| 15 | $-CH_3$ | -Br | 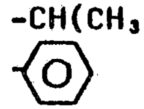 | O | O | O | |
| 16 | $-CH_3$ | -Br | $-CH-CO_2C_2H_5$<br>$CH_3$ | O | O | O | Sirup |
| 17 | $-CH_3$ | -Cl | $-CH_2-CO_2CH_3$ | S | O | O | Sirup |
| 18 | $-CH_3$ | -Cl | $-CH-CO_2C_2H_5$<br>$CH_3$ | S | O | O | Sirup |

Fortsetzung Tabelle 1

| Beispiel | $R_1$ | $R_2$ | $R_3$ | Z | X | Y | Fp °C] |
|---|---|---|---|---|---|---|---|
| 19 | $-CH_3$ | -Br | $-CH_3$ | S | O | O | |
| 20 | $-CH_3$ | -Br | $-CH_2-C{\equiv}CH$ | S | O | O | |
| 21 | $-CH_3$ | -Cl | $-CH(CH_3)_2$ | S | O | O | Sirup |
| 22 | $-CH_3$ | -Cl | $-CH_2-CO_2CH_2CH_2-OCH_3$ | S | O | O | |
| 23 | $-CH_3$ | -Cl | $-CH_2-CO_2CH-CO_2C_2H_5$<br>$CH_3$ | O | O | O | |
| 24 | $-CH_3$ | -Cl | $-CH_2-CO_2CH_2CF_3$ | O | O | O | |
| 25 | $-CH_3$ | -Cl | $-CH-CO_2CH_2CF_3$<br>$CH_3$ | O | O | O | |
| 26 | $-CH_3$ | -Cl | $-CF_2-CHF_2$ | O | O | O | |
| 27 | $-CH_3$ | -Cl | $-CF_2-CHFCl$ | O | O | O | |
| 28 | $-CH_3$ | -Cl | $-CF_2-CHF-CF_3$ | S | O | O | |
| 29 | $-CH_3$ | -Cl | $-CHF_2$ | O | O | O | |
| 30 | $-C_2H_5$ | -Cl | $-CH_3$ | O | O | O | |
| 31 | $-C_3H_7$ | -Br | $-CH_2-C{\equiv}CH$ | O | O | O | |
| 32 | $-CH_3$ | $-NO_2$ | $-CH_3$ | O | O | O | |
| 33 | $-CH_3$ | $-NO_2$ | $-CH_2-C{\equiv}CH$ | O | O | O | |
| 34 | $-CH_3$ | $-NO_2$ | $-CH_2-CO_2CH_3$ | S | O | O | |
| 35 | $-CH_3$ | $-NO_2$ | $-CH-CO_2C_2H_5$<br>$CH_3$ | S | O | O | |
| 36 | $-CH_3$ | -Br | $-CH_2-CH{=}CH_2$ | O | O | O | Sirup |
| 37 | $-CH_3$ | -Br | $-CH_2-$ 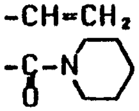 | O | O | O | |

Fortsetzung Tabelle 1

| Beispiel | $R_1$ | $R_2$ | $R_3$ | Z | X | Y | Fp [°C] |
|---|---|---|---|---|---|---|---|
| 38 | $-CH_3$ | -Br | 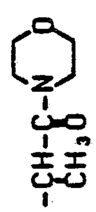 | O | O | O | Sirup |
| 40 | $-CH_3$ | -Br | 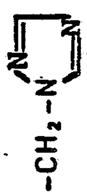 | O | O | O | |
| 41 | $-CH_3$ | -Cl | 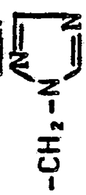 | O | O | O | |
| 42 | $-CH_3$ | -Cl | 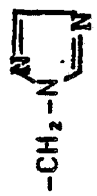 | S | O | O | |
| 43 | $-CH_3$ | -Br | 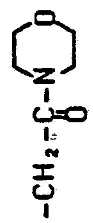 | O | O | O | Sirup |

## C. Biologische Beispiele

Die Schädigung der Unkrautpflanzen bzw. die Kulturpflanzenvertraglichkeit wurde gemäß einem Schlüssel bonitiert, in dem die Wirksamkeit durch Wertzahlen von 0-5 ausgedrückt ist. Dabei bedeutet:

0 = ohne Wirkung bzw. Schaden
1 = 0-20 % Wirkung bzw. Schaden

2 = 20-40 % Wirkung bzw. Schaden
3 = 40-60 % Wirkung bzw. Schaden
4 = 60-80 % Wirkung bzw. Schaden
5 = 80-100 % Wirkung bzw. Schaden

1. Unkrautwirkung im Vorauflauf

Samen bzw. Rhizomstücke von mono-und dikotylen Unkrautpflanzen wurden in Plastiktöpfen (0 = 9 cm) in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspensionen bzw. Emulsionen mit einer Wasseraufwandmenge von umgerechnet 600 l/ha in unterschiedlichen Dosierungen auf die Oberfläche der Abdeckerde appliziert.
Nach der Behandlung wurden die Töpfe im Gewächshaus aufgestellt und unter guten Wachstumsbedingungen für die Unkrautpflanzen gehalten (Temperatur 23 plus/minus 1 °C, relative Luftfeuchte 60-80 %).

Die optische Bonitur der Pflanzen bzw. Auflaufschäden erfolgte nach dem Auflaufen der Versuchspflanzen nach einer Versuchszeit von 3-4 Wochen im Vergleich zu unbehandelten Kontrollen.
Die Boniturwerte zeigen, daß die erfindungsgemäßen Verbindungen eine gute herbizide Vorauflaufwirksamkeit gegen ein breites Spektrum von Ungrösern und Unkräutern besitzen, s. Tabelle 2.

**Tabelle 2**

| Beispiel-Nr. | Dosis (kg a.i./ha) | herbizide Wirkung SIA | CRS | STM | ECG | LOM |
|---|---|---|---|---|---|---|
| 1 | 2,5 | 5 | 5 | 5 | 5 | 5 |
| 2 | 2,5 | 5 | 5 | 5 | 5 | 5 |
| 3 | 2,5 | 5 | 5 | 3 | 2 | 3 |
| 4 | 2,5 | 5 | 5 | 5 | 4 | 4 |
| 5 | 2,5 | 5 | 5 | 5 | 5 | 5 |
| 14 | 2,5 | 5 | 5 | 5 | 5 | 5 |
| 36 | 2,5 | 5 | 5 | 5 | 5 | 5 |
| 43 | 2,5 | 5 | 5 | 3 | 3 | 4 |

**Abkürzungen:**

SIA = Sinapis arvensis
CRS = Chrysanthemum segetum
STM = Stellaria media
ECG = Echinochloa crus-galli
LOM = Lolium multiflorum

2. Unkrautwirkung im Nachauflauf

Samen bzw. Rhizomstücke von mono-und dikotylen Unkräutern wurden in Plastiktöpfen (0 = 9 cm) in sandigem Lehmboden ausgelegt, mit Erde abgedeckt und im Gewächshaus unter guten Wachstumsbedingungen angezogen. Drei Wochen nach Aussaat wurden die Versuchspflanzen im Dreiblattstadium behandelt.

Die als Spritzpulver bzw. als Emulsionskonznetrate formulierten erfundungsgemäßen Verbindungen wurden in verschiedenen Dosierungen mit einer Wasseraufwandmenge von umgerechnet 600 l/ha auf die grünen Pflanzenteile gesprüht und nach ca. 3-4 Wochen Standzeit der Versuchspflanzen im Gewächshaus unter optimalen Wachstumsbedingungen (Temperatur 23 plus/minus 1 °C, relative Luftfeuchte 60-80 %) die Wirkung der Präparate optisch im Vergleich zu unbehandelten Kontrollen bonitiert.

Die erfindingsgemäßen Verbindungen weisen auch im Nachauflauf eine gute herbizide Wirksamkeit gegen ein breites Spektrum wirtschaftlich wichtiger Ungräser und Unkräuter auf, s. Tabelle 3.

**Tabelle 3**

| Beispiel-Nr. | Dosis (kg a.i./ha) | herbizide Wirkung SIA | CRS | STM | ECG | LOM |
|---|---|---|---|---|---|---|
| 1 | 2,5 | 5 | 5 | 5 | 5 | 5 |
| 2 | 2,5 | 5 | 5 | 5 | 5 | 4 |
| 3 | 2,5 | 5 | 5 | 5 | 3 | 2 |
| 4 | 2,5 | 5 | 5 | 5 | 5 | 5 |
| 5 | 2,5 | 5 | 5 | 5 | 4 | 5 |
| 14 | 2,5 | 5 | 5 | 5 | 5 | 5 |
| 36 | 2,5 | 5 | 5 | 5 | 4 | 2 |
| 43 | 2,5 | 5 | 5 | 5 | 2 | 3 |

**Abkürzungen:**

**SIA = Sinapis arvensis**
**CRS = Chrysanthemum segetum**
**STM = Stellaria media**
**ECG = Echinochloa crus-galli**
**LOM = Lolium multiflorum**

3. Kulturpflanzenverträglichkeit

In weiteren Versuchen im Gewächshaus wurden Samen einer größeren Anzahl von Kulturpflanzen in sandigem Lehmboden ausgelegt und mit Erde abgedeckt.

Ein Teil der Töpfe wurde sofort wie unter 1. beschrieben behandelt; die übrigen wurden im Gewächshaus aufgestellt, bis die Pflanzen zwei bis drei echte Blätter entwickelt hatten und dann mit den erfindungsgemäßen Substanzen in unterschiedlichen Dosierungen, wie unter 2. beschrieben, besprüht.

Vier bis fünf Wochen nach der Applikation und Standzeit im Gewächshaus wurde mittels optischer Bonitur festgestellt, daß die erfindungsgemäßen Verbindungen zweikeimblättrige Kulturen wie z.B. Soja, Baumwolle, Raps, Zuckerrüben und Kartoffeln im Vor-und Nachauflaufverfahren selbst bei hohen Wirkstoffdosierungen ungeschädigt ließen. Einige Substanzen schonten darüber hinaus auch Gramineen-Kulturen wie z.B. Gerste, Sorghum, Mais, Weizen oder Reis. Die Verbindungen der Formel I weisen somit eine hohe Selektivität bei Anwendung zur Bekämpfung von unerwünschtem Pflanzenwuchs in landwirtschaftlich wichtigen Kulturen auf.

## Ansprüche

1. Verbindungen der Formel I

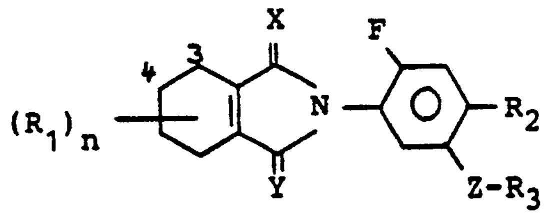

(I)

worin
X,Y,Z unabhängig voneinander O oder S,
$R_1$ $(C_1-C_4)$Alkyl,
$R_2$ Fluor, Chlor, Brom oder $NO_2$
$R_3$ $(C_1-C_4)$Alkyl, Halogen$(C_1-C_4)$alkyl, $(C_3-C_6)$Cycloalkyl, $(C_3-C_4)$Alkenyl, $(C_3-C_4)$Alkinyl, Cyano$(C_1-C_4)$alkyl, $(C_1-C_4)$Alkoxy-$(C_1-C_4)$alkyl, $(C_1-C_4)$Alkylthio$(C_1-C_4)$alkyl, $(C_1-C_4)$Alkoxycarbonyl$(C_1-C_4)$alkyl, wobei der Alkoxyteil bis zu 3-fach durch Halogen substituiert sein kann,
$(C_1-C_4)$Alkylthiocarbonyl$(C_1-C_4)$alkyl, $(C_1-C_4)$Alkoxythiocarbonyl$(C_1-C_4)$alkyl, $(C_1-C_4)$Alkoxy$(C_1-C_4)$-alkoxycarbonyl$(C_1-C_4)$alkyl, $(C_1-C_4)$Alkoxycarbonyl$(C_1-C_4)$alkoxycarbonyl-$(C_1-C_4)$alkyl,
Phenyl oder Phenyl$(C_1-C_4)$alkyl, wobei der Phenylring der beiden Reste bis zu 3-fach durch Halogen, $(C_1-C_4)$Alkyl, $(C_1-C_4)$Alkoxy, $(C_1-C_4)$-Alkoxycarbonyl, $CF_3$, $NO_2$ oder CN substituiert sein kann, oder
einen Rest der Formeln

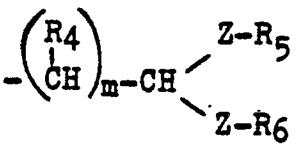

,

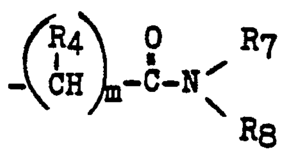

oder

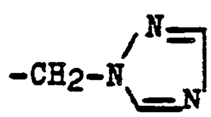

$R_4$ Wasserstoff oder $(C_1-C_4)$Alkyl,
$R_5$, $R_6$ $(C_1-C_4)$Alkyl oder beide Reste zusammen eine Ethylen-oder Propylen-Kette,
$R_7$, $R_8$ unabhängig voneinander Wasserstoff, $(C_1-C_4)$Alkyl, Phenyl oder Benzyl, die beide im Phenylteil bis zu zweifach durch $(C_1-C_4)$Alkyl, Halogen, $(C_1-C_4)$Alkoxy, $CF_3$, $NO_2$ oder CN substituiert sein können; oder $R_7$ und $R_8$ bilden zusammen mit dem sie verknüpfenden N-Atom einen 5-oder 6-gliedrigen gesättigten Heterocyclus, der als Ringglied auch ein Sauerstoffatom oder eine Gruppe $N-R^4$ enthalten kann und der bis zu zweifach durch $(C_1-C_4)$Alkyl an den $CH_2$-Ringgliedern substituiert sein kann,
n 1 oder 2 und
m 1, 2 oder 3
bedeuten.

2. Verbindungen der Formel I gemäß Anspruch 1, worin
X, Y Sauerstoff,
$R_1$ in Position 4 orientiert ist und Methyl,
$R_2$ Chlor oder Brom,
$R_3$ $(C_1-C_4)$Alkyl, Halogen$(C_1-C_4)$-alkyl, $(C_3-C_4)$Alkenyl; $(C_3-C_4)$Alkinyl; $(C_1-C_4)$Alkoxycarbonyl$(C_1-C_4)$alkyl; Cyano$(C_1-C_4)$Alkyl; Benzyl, das bis zu zweifach durch Halogen, $(C_1-C_4)$Alkyl, $(C_1-C_4)$Alkoxy oder $CF_3$ substituiert ist oder ein Rest der Formel

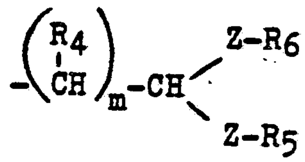,
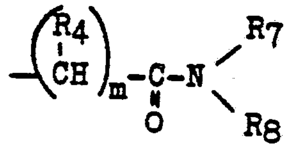 oder

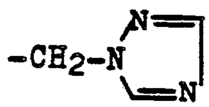

n = 1 und m = 1 oder 2 bedeuten.

3. Verfahren zur Herstellung von Verbindungen der Formel I gemäß Anspruch 1 oder 2, dadurch gekennzeichnet, daß man

a) eine Verbindung der Formel II mit einer Verbindung der Formel III

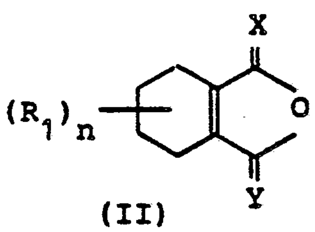

(II)

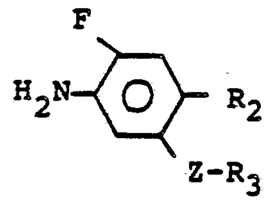

(III)

in Gegenwart von Wasser oder einem organischen Lösungsmittel, oder

b) eine Verbindung der Formel IV

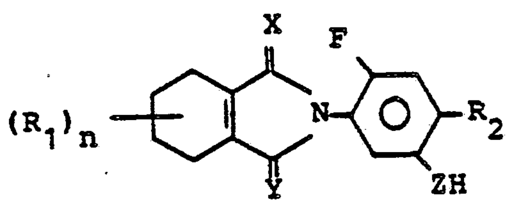

$R_3$-Hal
(V)

mit einer Verbindung der Formel (V) in Gegenwart einer Base und eines organischen Lösungsmittels umsetzt.

4. Verwendung von Verbindungen der Formel I gemäß Anspruch 1 oder 2 als Herbizide.

5. Herbizide Mittel, dadurch gekennzeichnet, daß sie eine Verbindung der Formel I von Anspruch 1 oder 2 enthalten.

6. Verfahren zur Bekämpfung von unerwünschten Pflanzen, dadurch gekennzeichnet, daß man eine wirksame Menge einer Verbindung der Formel I von Anspruch 1 oder 2 auf die zu behandelnden Anbauflächen oder die Pflanzen aufbringt.

Patentansprüche für den folgenden Vertragsstaat: ES

1. Verfahren zur Herstellung der Verbindung der Formel I

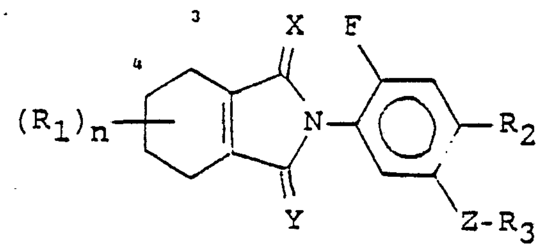

(I)

worin
X,Y,Z unabhängig voneinander O oder S,
$R_1$ $(C_1-C_4)$Alkyl,
$R_2$ Fluor, Chlor, Brom oder $NO_2$
$R_3$ $(C_1-C_4)$Alkyl, Halogen$(C_1-C_4)$alkyl, $(C_3-C_6)$Cycloalkyl, $(C_3-C_4)$Alkenyl, $(C_3-C_4)$Alkinyl, Cyano$(C_1-C_4)$alkyl, $(C_1-C_4)$Alkoxy-$(C_1-C_4)$alkyl, $(C_1-C_4)$Alkylthio$(C_1-C_4)$alkyl, $(C_1-C_4)$Alkoxycarbonyl$(C_1-C_4)$Alkyl, wobei der Alkoxyteil bis zu 3-fach durch Halogen substituiert sein kann,
$(C_1-C_4)$Alkylthiocarbonyl$(C_1-C_4)$alkyl, $(C_1-C_4)$Alkoxythiocarbonyl$(C_1-C_4)$alkyl, $(C_1-C_4)$Alkoxy$(C_1-C_4)$-alkoxycarbonyl$(C_1-C_4)$alkyl, $(C_1-C_4)$Alkoxycarbonyl$(C_1-C_4)$alkoxycarbonyl-$(C_1-C_4)$alkyl,
Phenyl oder Phenyl$(C_1-C_4)$alkyl, wobei der Phenylring der beiden Reste bis zu 3-fach durch Halogen, $(C_1-C_4)$Alkyl, $(C_1-C_4)$Alkoxy, $(C_1-C_4)$-Alkoxycarbonyl, $CF_3$, $NO_2$ oder CN substituiert sein kann, oder
einen Rest der Formeln

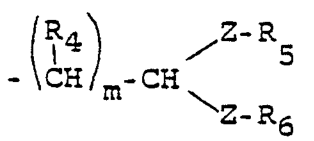

,

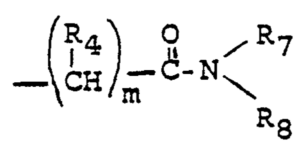

oder

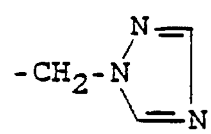

$R_4$ Wasserstoff oder $(C_1-C_4)$Alkyl,
$R_5$, $R_6$ $(C_1-C_4)$Alkyl oder beide Reste zusammen eine Ethylen-oder Propylen-Kette,
$R_7$, $R_8$ unabhängig voneinander Wasserstoff, $(C_1-C_4)$Alkyl, Phenyl oder Benzyl, die beide im Phenylteil bis zu zweifach durch $(C_1-C_4)$Alkyl, Halogen, $(C_1-C_4)$Alkoxy, $CF_3$, $NO_2$ oder CN substituiert sein können; oder $R_7$ und $R_8$ bilden zusammen mit dem sie verknüpfenden N-Atom einen 5-oder 6-gliedrigen gesättigten Heterocyclus, der als Ringglied auch ein Sauerstoffatom oder eine Gruppe $>N-R^4$ enthalten kann und der bis zu zweifach durch $(C_1-C_4)$Alkyl an den $CH_2$-Ringgliedern substituiert sein kann,
n 1 oder 2 und
m 1, 2 oder 3
bedeuten, dadurch gekennzeichnet, daß man
a) eine Verbindung der Formel (II) mit einer Verbindung der Formel (III)

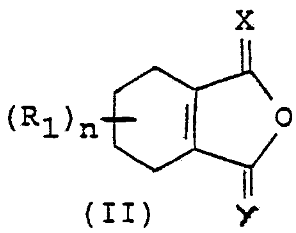
(II)

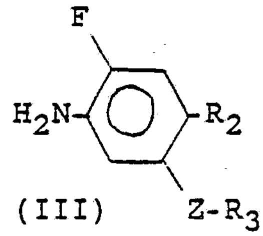
(III)

in Gegenwart von Wasser oder einem organischen Lösungsmittel, oder

b) eine Verbindung der Formel (IV)

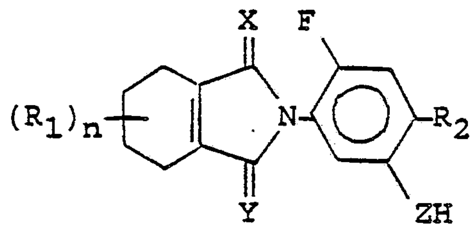

$R_3$-Hal

(V)

mit einer Verbindung der Formel (V) in Gegenwart einer Base und eines organischen Lösungsmittels umsetzt.

2. Verbindungen der Formel I gemäß Anspruch 1, worin X, Y Sauerstoff,
$R_1$ in Position 4 orientiert ist und Methyl,
$R_2$ Chlor oder Brom,
$R_3$ $(C_1$-$C_4)$Alkyl, Halogen$(C_1$-$C_4)$-alkyl, $(C_3$-$C_4)$Alkenyl; $(C_3$-$C_4)$Alkinyl; $(C_1$-$C_4)$Alkoxycarbonyl$(C_1$-$C_4)$alkyl; Cyano$(C_1$-$C_4)$Alkyl; Benzyl, das bis zu zweifach durch Halogen, $(C_1$-$C_4)$Alkyl, $(C_1$-$C_4)$Alkoxy oder $CF_3$ substituiert ist oder ein Rest der Formel

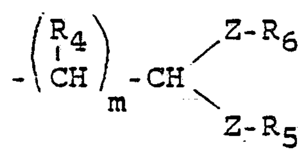
,
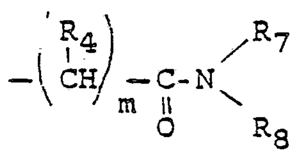
oder

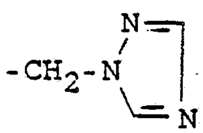

n = 1 und m = 1 oder 2 bedeuten.

3. Verfahren zur Bekämpfung von unerwünschten Pflanzen, dadurch gekennzeichnet, daß man eine wirksame Menge einer Verbindung der Formel I von Anspruch 1 oder 2 auf die zu behandelnden Anbauflächen oder die Pflanzen aufbringt.

4. Verwendung von Verbindungen der nach Anspruch 1 oder 2 definierten Formel I als Herbizide.

5. Herbizide Mittel, dadurch gekennzeichnet, daß sie eine Verbindung der nach Anspruch 1 oder 2 definierten Formel I in Kombination mit üblichen Hilfsstoffen für Wirkstoffzubereitungen enthalten.

6. Herbizide Mittel nach Anspruch 5, dadurch gekennzeichnet, daß sie als Spritzpulver, emulgierbare Konzentrate, versprühbare Lösungen, Stäubemittel, Beizmittel, Dispersionen, Granulate oder Mikrogranulate in den üblichen Zubereitungen vorliegend.

Europäisches Patentamt

# EUROPÄISCHER RECHERCHENBERICHT

Nummer der Anmeldung

EP 88106619.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A,D | DE - A1 - 3 109 035 (NIPPON KAYAKU K.K.)<br>* Patentansprüche 1,22,26; Seiten 13,14 *<br>-- | 1,3-6 | C 07 D 209/48<br>A 01 N 37/32 |
| A | EP - A2 - 0 216 243 (HOECHST AKTIENGESELLSCHAFT)<br>* Patentansprüche 1,4-6 *<br>-- | 1,3-6 | |
| A | EP - A1 - 0 207 894 (CIBA-GEIGY AG)<br>* Patentansprüche 1,35,39,40 *<br>-- | 1,3-6 | |
| A | CHEMICAL ABSTRACTS, Band 104, Nr. 1, 6. Jänner 1986, Columbus, Ohio, USA<br>MITSUBISHI CHEMICAL INDUSTRIES CO., LTD. "Herbicidal tetrahydrophthalimides"<br>Seite 528, Spalte 2, Zusammenfassung-Nr. 5 770b<br>& Jpn. Kokai Tokkyo Koho JP 60,123,469<br>-- | 1,3-6 | RECHERCHIERTE SACHGEBIETE (Int. Cl.4)<br>C 07 D 209/00 |

Der vorliegende Recherchenbericht wurde für alle Patentansprüche erstellt.

| Recherchenort | Abschlußdatum der Recherche | Prüfer |
|---|---|---|
| WIEN | 14-07-1988 | HEIN |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung

EP 88106619.5

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A | CHEMICAL ABSTRACTS, Band 106, Nr. 5, 2. Februar 1987, Columbus, Ohio, USA<br>SATO, RYO et al. "Synergistic herbicidal compositions containing an isoindole and a triazine derivative"<br>Seite 169, Spalten1/2, Zusammenfassung-Nr. 28 841f<br>& EP-A-0 198 298<br>-- | 1,3-6 | |
| A | CHEMICAL ABSTRACTS, Band 105, Nr. 17, 27. Oktober 1986, Columbus, Ohio, USA<br>MORITA et al. "Wide-spectrum herbicide composition"<br>Seite 263, Spalten1/2, Zusammenfassung-Nr. 148 204b<br>& Jpn. Kokai Tokkyo Koho JP 61,103,804<br>---- | 1,3-6 | RECHERCHIERTE SACHGEBIETE (Int. Cl.4) |

Der vorliegende Recherchenbericht wurde für alle Patentansprüche erstellt.

| Recherchenort | Abschlußdatum der Recherche | Prüfer |
|---|---|---|
| WIEN | 14-07-1988 | HEIN |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument